# FASCICULE DE BREVET EUROPEEN

(11) **EP 1 442 737 B1**
(45) Date de publication et mention de la délivrance du brevet: **29.03.2006**
(21) Numéro de dépôt: 03293162.8
(22) Date de dépôt: 15.12.2003
(51) Int. Cl.: A61Q 19/00, A61Q 5/00, A61K 8/37, C07C 59/13, C07C 235/74

(54) **Utilisation d'un dérivé d'acide (dihydro)jasmonique pour le traitement des peaux sèches**
Verwendung eines (Dihydro)jasmonsäure-Derivativen zur Behandlung von trockener Haut
Use of a (dihydro)jasmonic acid derivative for dry skin treatment

(30) Priorité: 31.01.2003 FR 0301146
(43) Date de publication de la demande: 04.08.2004
(73) Titulaire: L'OREAL, 75008 Paris (FR)
(72) Inventeur: Dalko, Maria, 91190 Gif S/Yvette (FR)
(74) Mandataire: Kromer, Christophe

(56) Documents cités:
- EP-A- 1 031 554
- EP-A- 1 333 021
- EP-A- 1 333 022
- WO-A-01/05388
- DATABASE WPI Section Ch, Week 199819 Derwent Publications Ltd., London, GB; Class B05, AN 1998-212696 XP002257101 & JP 10 059829 A (NOEVIR KK) 3 mars 1998 (1998-03-03)
- PATENT ABSTRACTS OF JAPAN vol. 2000, no. 24, 11 mai 2001 (2001-05-11) & JP 2001 199832 A (POLA CHEM IND INC), 24 juillet 2001 (2001-07-24)
- PATENT ABSTRACTS OF JAPAN vol. 1999, no. 08, 30 juin 1999 (1999-06-30) & JP 11 079948 A (NOEVIR CO LTD), 23 mars 1999 (1999-03-23)
- DATABASE WPI Section Ch, Week 199815 Derwent Publications Ltd., London, GB; Class B05, AN 1998-163664 XP002257102 & JP 10 029935 A (NOEVIR KK) 3 février 1998 (1998-02-03)
- DATABASE BEILSTEIN [Online] BEILSTEIN INSTITUTE FOR ORGANIC CHEMISTRY, FRANKFURT-MAIN, DE; XP002282164 extrait de STN Database accession no. 2767106
- DATABASE BEILSTEIN [Online] BEILSTEIN INSTITUTE FOR ORGANIC CHEMISTRY, FRANKFURT-MAIN, DE; XP002282165 extrait de STN Database accession no. 8283269
- DATABASE BEILSTEIN [Online] BEILSTEIN INSTITUTE FOR ORGANIC CHEMISTRY, FRANKFURT-MAIN, DE; XP002282166 extrait de STN Database accession no. 8283270
- DATABASE BEILSTEIN [Online] BEILSTEIN INSTITUTE FOR ORGANIC CHEMISTRY, FRANKFURT-MAIN, DE; XP002282167 extrait de STN Database accession no. 8283363
- DATABASE BEILSTEIN [Online] BEILSTEIN INSTITUTE FOR ORGANIC CHEMISTRY, FRANKFURT-MAIN, DE; XP002282168 extrait de STN Database accession no. 8286879
- DATABASE BEILSTEIN [Online] BEILSTEIN INSTITUTE FOR ORGANIC CHEMISTRY, FRANKFURT-MAIN, DE; XP002282169 extrait de STN Database accession no. 3235024
- DATABASE BEILSTEIN [Online] BEILSTEIN INSTITUTE FOR ORGANIC CHEMISTRY, FRANKFURT-MAIN, DE; XP002282170 extrait de STN Database accession no. 3235025
- DATABASE BEILSTEIN [Online] BEILSTEIN INSTITUTE FOR ORGANIC CHEMISTRY, FRANKFURT-MAIN, DE; XP002282171 extrait de STN Database accession no. 3090159
- DATABASE BEILSTEIN [Online] BEILSTEIN INSTITUTE FOR ORGANIC CHEMISTRY, FRANKFURT-MAIN, DE; XP002282172 extrait de STN Database accession no. 3289199
- DATABASE BEILSTEIN [Online] BEILSTEIN INSTITUTE FOR ORGANIC CHEMISTRY, FRANKFURT-MAIN, DE; XP002282173 extrait de STN Database accession no. 4182970
- DATABASE BEILSTEIN [Online] BEILSTEIN INSTITUTE FOR ORGANIC CHEMISTRY, FRANKFURT-MAIN, DE; XP002282174 extrait de STN Database accession no. 4391401
- DATABASE BEILSTEIN [Online] BEILSTEIN INSTITUTE FOR ORGANIC CHEMISTRY, FRANKFURT-MAIN, DE; XP002282175 extrait de STN Database accession no. 4397972
- DATABASE BEILSTEIN [Online] BEILSTEIN INSTITUTE FOR ORGANIC CHEMISTRY, FRANKFURT-MAIN, DE; XP002282176 extrait de STN Database accession no. 4989373
- DATABASE BEILSTEIN [Online] BEILSTEIN INSTITUTE FOR ORGANIC CHEMISTRY, FRANKFURT-MAIN, DE; XP002282177 extrait de SN Database accession no. 5500739
- DATABASE BEILSTEIN [Online] BEILSTEIN INSTITUTE FOR ORGANIC CHEMISTRY, FRANKFURT-MAIN, DE; XP002282178 extrait de STN Database accession no. 6652596
- DATABASE BEILSTEIN [Online] BEILSTEIN INSTITUTE FOR ORGANIC CHEMISTRY, FRANKFURT-MAIN, DE; XP002282179 extrait de STN Database accession no. 7128041

## Description

La présente invention concerne un procédé cosmétique de traitement de la peau sèche ou du cuir chevelu sec d'origine non inflammatoire, en particulier d'une femme ménopausée, comprenant l'application topique sur la peau ou le cuir chevelu, d'une composition renfermant, dans un milieu physiologiquement acceptable, au moins un dérivé d'acide (dihydro)jasmonique.

De nombreuses femmes à partir de trente-cinq ans, et plus particulièrement après la ménopause, se plaignent fréquemment du dessèchement de leur peau, et des manifestations d'inconfort ou inesthétiques qui en résultent (desquamation, teint terne, atonie cutanée). Or, ce dessèchement est dû, entre autres, comme on le sait maintenant, à une diminution de la production de sébum avec l'âge.

Le sébum est le produit naturel de la glande sébacée qui, conjointement à la sueur produite par les glandes eccrines ou aprocrines, constitue un hydratant naturel de l'épiderme. Il est constitué essentiellement d'un mélange plus ou moins complexe de lipides. Classiquement, la glande sébacée produit du squalène, des triglycérides, des cires aliphatiques, des cires de cholestérol et éventuellement du cholestérol libre (Stewart, M. E., Semin. Dermatol. 11, 100-105 (1992)). L'action des lipases bactériennes convertit une part variable, et quelquefois l'intégralité, des triglycérides en acides gras libres.

Le sébocyte constitue la cellule compétente de la glande sébacée. La production de sébum est associée au programme de différenciation terminale de cette cellule. Durant cette différenciation, l'activité métabolique du sébocyte est essentiellement axée sur la biosynthèse des lipides (la lipogénèse) et plus précisément sur la néosyntèse d'acides gras et de squalène.

Un composé permettant de stimuler la production des lipides constituant le sébum, par les cellules de la glande sébacée (les sébocytes), serait donc d'un intérêt certain pour le traitement des peaux sèches oligoséborrhéiques, caractéristiques des femmes ménopausées, c'est-à-dire des peaux présentant un taux de sébum inférieur à 100 µg/cm² au niveau du front à la mi-journée.

A cette fin, il a été proposé dans le brevet US-4,496,556 d'utiliser la DHEA, un stéroïde sécrété par les glandes surrénales, ou ses esters, administrés par voie topique, pour augmenter la production de sébum.

Toutefois, pour un certain nombre de raisons légales et toxicologiques, il n'est pas toujours possible d'utiliser ce type de composés dans le domaine cosmétique. En outre, son efficacité n'est pas suffisante sur les peaux oligoséborrhéiques. II subsiste donc le besoin de disposer de composés cosmétiquement acceptables, permettant de stimuler efficacement la fonction sébacée en vue de traiter les peaux sèches oligoséborrhéiques.

La Demanderesse a maintenant découvert avec étonnement que certains dérivés d'acide (dihydro)jasmonique permettaient de satisfaire ce besoin.

Il est connu d'utiliser des dérivés d'acide (dihydro)jasmonique analogues de prostaglandines comme agents anti-inflammatoires, en vue de traiter des troubles des sécrétions, en particulier salivaires et lacrymales, mais également les peaux sèches, d'origine inflammatoire (WO 01/05388). Il est également connu de la demande JP2001-199 832 d'utiliser le dihydrojasmonate de méthyle comme desquamant par activation des protéases de la couche cornée, en particulier dans le traitement des peaux sèches. Enfin, on connaît des toniques capillaires comprenant des dérivés d'acide jasmonique et qui sont notamment capables de retenir l'humidité du cuir chevelu lorsqu'ils sont incorporés dans la bicouche lipidique d'une vésicule telle qu'un liposome (JP-10 059 829).

Toutefois, à la connaissance de la Demanderesse, il n'a encore jamais été suggéré que les dérivés d'acide (dihydro)jasmonique pouvaient être utiles dans le traitement de la peau et du cuir chevelu secs de femmes ménopausées, qui sont dus à une sécrétion insuffisante de sébum d'origine hormonale qui ne peut être traitée par des anti-inflammatoires et/ou avec des desquamants.

Au contraire, les demandes JP-11 079 948 et JP-10 029 235 divulguent l'effet anti-androgène (mis en évidence sur des cellules cancéreuses de souris) de compositions contenant des dérivés d'acide dihydrojasmonique et éventuellement des extraits botaniques et suggère d'utiliser ces compositions dans le traitement de la séborrhée, qui est pour l'essentiel androgéno-dépendante.

Or, la Demanderesse a mis en évidence, contre toute attente, un effet positif des dérivés d'acide (dihydro)jasmonique sur la production de sébum.

La présente invention a donc pour objet un procédé cosmétique de traitement de la peau sèche ou du cuir chevelu sec d'origine non inflammatoire, d'une femme ménopausée, comprenant l'application topique sur la peau ou le cuir chevelu, d'une composition renfermant, dans un milieu physiologiquement acceptable, au moins un dérivé d'acide (dihydro)jasmonique choisi parmi les composés de formule (I) : 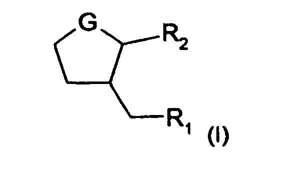 dans laquelle :
G désigne un groupe choisi parmi les groupes : C=O ; CH-OHR₁est un radical -COOR où R est un atome d'hydrogène ou un radical hydrocarboné comportant de 1 à 6 atomes de carbone, linéaire ou ramifié, saturé ou insaturé, non substitué, de préférence un radical méthyle ; R₂ est un radical hydrocarboné, linéaire ou ramifié, saturé ou insaturé, non substitué, comportant 5 atomes de carbone, de préférence un radical n-pentyle.

Un exemple préféré d'un tel composé est le dihydrojasmonate de méthyle (ou l'un de ses isomères, stéréoisomères et sels) qui est notamment disponible dans le commerce auprès de la société FIRMENICH sous la référence commerciale HEDIONE 964898.

D'autres composés utilisables dans la présente invention sont notamment le jasmonate de méthyle qui peut être préparé comme décrit dans KIYOTA et al., Lipase-catalyzed préparation of both enantiomers of methyl jasmonate, *Tetrahedron : assymetry,* Vol. 12, n° 7, pp. 1035-1038 (2001) ; et les composés dans lesquels G désigne un groupe -CH-OH, en particulier les composés dans lesquels R₁ = COOCH₃ et R₂ est un radical n-pentyle ou 2,3-pentènyle.

Les composés de formule (I) peuvent être préparés selon un procédé analogue à ceux donnés dans les exemples ci-après.

La présente invention a également pour objet l'utilisation cosmétique d'au moins un dérivé d'acide (dihydro)jasmonique, tel que défini précédemment, en tant qu'agent pour le traitement de la peau sèche ou du cuir chevelu sec d'origine non inflammatoire, de femmes ménopausées.

La composition pour le procédé selon l'invention est de préférence destinée à être appliquée sur des personnes présentant une sécrétion de sébum insuffisante, telles des femmes ménopausées, qui ont généralement un taux de sébum inférieur à 100 µg/cm² au niveau du front caractéristique d'une peau et/ou d'un cuir chevelu oligoséborrhéiques.

La composition pour le procédé sélon l'invention permet de restaurer la production de sébum par les sébocytes et, par là-même, d'améliorer le confort des peaux et cuirs chevelus secs. Elle permet également de lutter contre l'aspect terne et/ou atone de la peau et/ou des cheveux, conséquences de leur dessèchement.

La quantité de dérivé d'acide (dihydro)jasmonique utilisable selon l'invention est bien entendu fonction de l'effet recherché et peut donc varier dans une large mesure. Pour donner un ordre de grandeur, on peut utiliser le dérivé d'acide (dihydro)jasmonique en une quantité représentant de 0,01 % à 20% du poids total de la composition, de préférence en une quantité représentant de 0,1% à 10% du poids total de la composition et, plus préférentiellement, en une quantité représentant de 0,5% à 5% du poids total de la composition.

La composition pour le procédé selon l'invention est généralement adaptée à une application topique sur la peau et/ou le cuir chevelu et elle contient donc un milieu physiologiquement acceptable, c'est-à-dire compatible avec la peau, ses phanères (cils, ongles, cheveux) et/ou les muqueuses.

Cette composition peut se présenter sous toutes les formes galéniques normalement utilisées dans les domaines cosmétique et dermatologique, et elle peut être notamment sous forme d'une solution éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau. Elle peut aussi être utilisée comme shampooing ou après-shampooing.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des dérivés d'acide (dihydro)jasmonique selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 à 30% en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Selon une forme d'exécution préférée de l'invention, les huiles et autres corps gras comprennent ceux naturellement présents dans le sébum, tels que le squalène, les triglycérides et les cires de cholestérol.

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, le palmitate de dextrine et la silice hydrophobe.

Comme actifs, il sera avantageux d'introduire dans la composition utilisée selon l'invention au moins un composé choisi parmi : les agents desquamants ; les agents hydratants ; les agents anti-inflammatoires ou apaisants ; les agents stimulant la prolifération et/ou la différenciation des kératinocytes ; les agents anti-chute des cheveux ; les agents anti-pelliculaires ; et les agents anti-bactériens.

En effet, la stimulation de la séborrhée par les dérivés d'acide (dihydro)jasmonique selon l'invention peut, chez certaines personnes, fournir un terrain de prolifération pour la microflore résidente de l'ostium folliculaire *(Propionibacterium acnes* en particulier), provoquant ainsi une hydrolyse importante des triglycérides du sébum en acides gras libres et la réduction des insaturations des acides gras poly-insaturés (acide linoléique en particulier). Ces deux phénomènes peuvent concourir à une kératinisation de l'infundibulum et à la formation d'un micro-comédon. Celui-ci peut dégénérer en comédon, bouchant et dilatant le pore de façon inesthétique. A un stade plus avancé, ce bouchon peut diverger vers une lésion acnéique inflammatoire.

L'ajout d'agents desquamants ou stimulant la prolifération ou la différenciation des kératinocytes à la composition selon l'invention permettent d'éviter la formation de ces comédons. De même, des agents anti-bactériens ou bactériostatiques permettent, en modérant la prolifération de la microflore résidente, d'obtenir le même effet.

En outre, les agents hydratants peuvent compléter l'effet obtenu à l'aide des dérivés d'acide (dihydro)jasmonique selon l'invention, et les agents apaisants sont utiles pour améliorer le confort des peaux sèches oligoséborrhéiques.

Enfin, l'utilisation d'agents anti-chute ou anti-pelliculaires est utile dans le cas où la composition selon l'invention est destinée au traitement des cuirs chevelus secs.

L'invention sera maintenant illustrée par les exemples non limitatifs suivants. Dans ces exemples, les quantités sont indiquées en pourcentage pondéral.

### EXEMPLES

### Exemple 1 : Procédé de préparation des dérivés alcools de l'acide (dihydro)jasmonique

Le procédé ci-dessous peut être utilisé, par analogie, pour synthétiser d'autres dérivés de l'acide jasmonique de formule (I) dans laquelle G = CH-OH.

### 1/ étape 1 : synthèse de l'acide jasmonique (+/-) ou acide (1R,2R) 3-oxo-2-[(2Z)-2-pentenyl]- cyclopentaneacétique (+/-)

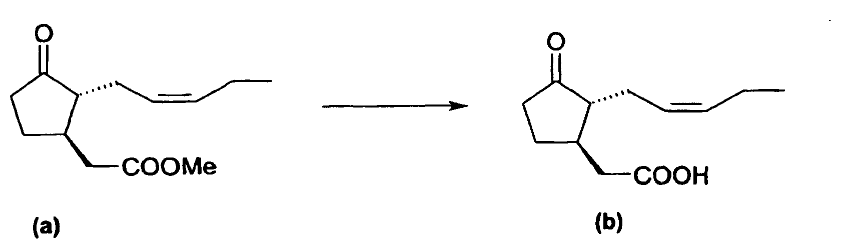

Dans un tricol de 250 ml muni d'un réfrigérant, d'un thermomètre et d'une agitation magnétique, on dissout 15 g (66,9 mmol) de (+/-) jasmonate de méthyle **(a)** dans 150 ml d'acétone. On additionne lentement 10 ml de solution aqueuse de soude (5,35 g, 133,7 mmol). Le mélange est agité pendant 5 heures à température ambiante. L'acétone est alors évaporée sous vide puis la phase aqueuse résiduelle est lavée par de l'acétate d'éthyle (2 x 30 ml). La phase aqueuse est acidifiée par de l'acide chlorhydrique jusqu'à pH=2, puis extraite par du dichlorométhane (3 x 30 ml).
La phase organique est séchée sur sulfate de sodium, filtrée sur papier filtre puis concentrée. L'huile marron clair obtenue est séchée sous vide.

On obtient 13,6 g d'acide jasmonique (+/-) **(b)** soit un rendement de 97%. Le spectre RMN ¹H et le spectre de masse (ionisation négative) sont conformes à la structure attendue.

### 2/ étape 2 synthèse de l'acide (1R, 2R) 3-hydroxy-2-[(2Z)-2-pentenyl]-cyclopentaneacétique (+/-)

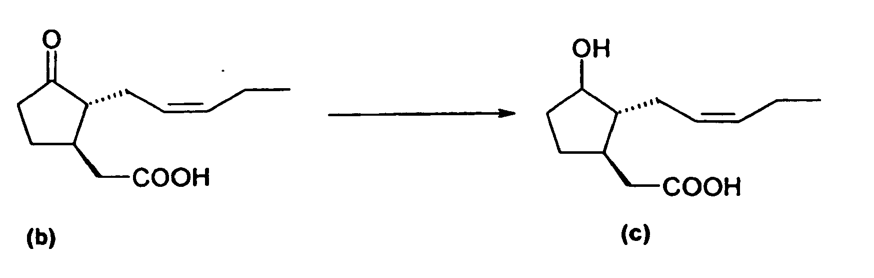

Dans un tricol de 50 ml muni d'un réfrigérant, d'un thermomètre et d'une agitation magnétique, on dissout 1 g (4,8 mmol) d'acide jasmonique (+/-) **(b)** dans 15 ml d'éthanol absolu. On ajoute 430 mg (11,4 mmol) de borohydrure de sodium NaBH₄. Le mélange est agité pendant 4 heures à 50°C. Une fois la réaction terminée, on ajoute lentement 5 ml d'eau. Le précipité formé est filtré. Le filtrat est acidifié par de l'acide chlorhydrique jusqu'à pH=5 puis extrait par de l'acétate d'éthyle (3 x 30 ml). La phase organique est séchée sur sulfate de sodium, filtrée sur papier filtre puis concentrée. L'huile incolore obtenue est purifiée par chromatographie sur gel de silice (éluant : dichlorométhane/méthanol). L'huile incolore obtenue est séchée sous vide.

On obtient 400 mg du composé **(c)** recherché, soit un rendement de 40%. Le spectre RMN ¹H est conforme à la structure attendue.

### Exemple 2 : Procédé de préparation des alkylesters de l'acide (dihydro)jasmonique

Le procédé ci-dessous peut être utilisé, par analogie, pour préparer d'autres composés de formule (I) dans lesquels R₁ est un radical alcoxycarbonyle et G est soit un groupe C=O, soit un groupe -CH-OR.

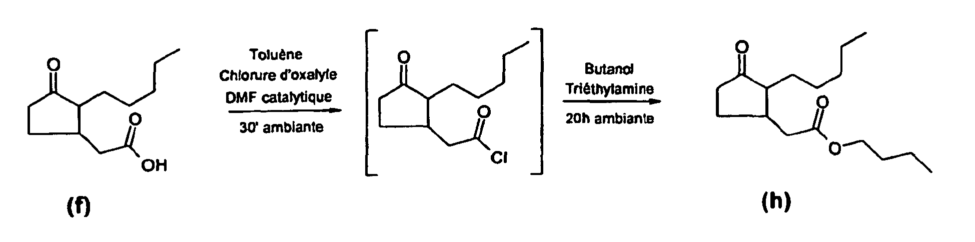

On active d'abord l'acide dihydrojasmonique **(f)** par 2,1 équivalents de chlorure d'oxalyle, dans le toluène, avec une catalyse au DMF. Après 30 minutes, on considère que la réaction est complète. Le milieu est alors concentré au rotavapeur. Le chlorure d'acide précédemment préparé est solubilisé dans du butanol en présence de 2,1 équivalents de triéthylamine. On laisse réagir 20 heures à température ambiante avant traitement et purification sur colonne de silice.

On récupère ainsi 400 mg de produit **(h)** (rendement : 63%). Le spectre de masse et la RMN sont conformes.

### Exemple 3 : Mise en évidence de l'activité du dihydrojasmonate de méthyle

Le dihydrojasmonate de méthyle (fourni par BEDOUKIAN) a été testé sur un modèle de sébocytes humains immortalisés en culture, issus de la lignée SZ95 décrite dans Zouboulis, C.C., Seltmann, H., Neitzel, H. & Orfanos, C.E., Establishment and Characterization of an lmmortalized Human Sebaceous Gland Cell Line, J. Invest. Dermatol., 113, 1011-1020 (1999).

Le test a consisté à mesurer la quantité de lipides produite par les sébocytes de la lignée (à confluence), en présence ou non d'agent actif dilué dans le DMSO, de telle sorte que la quantité finale de DMSO dans le milieu de culture soit 0,1 % et la quantité de dihydrojasmonate de méthyle de 10⁻⁴ M. Après 48 heures de traitement, les cellules adhérentes sont traitées par du Rouge de Nile (1µg/ml). Le contenu en lipides est ensuite quantifié par mesure de la fluorescence du colorant (deux couples d'excitation/émission : 485-540nm pour les lipides neutres et 540-620 nm pour les lipides non neutres). Les résultats sont donnés pour les lipides totaux (combinaison des deux mesures).

L'expérience est réalisée en sixplicate (produits dosés et témoin) en plaque de 96 puits et renouvelée quatre fois.

Pour les échantillons traités par le dihydrojasmonate de méthyle, la quantité de lipides neutres synthétisés par les sébocytes est augmentée de 98% par rapport au témoin non traité (p<0,001).

Ce composé induit donc une augmentation très significative de la lipogénèse sébocytaire.

Des tests de prolifération (MUH) et de viabilité cellulaire (LDH) ont permis de vérifier en parallèle que les effets obtenus n'étaient pas liés à une modification de ces paramètres biologiques.

### Exemple 4 : Composition cosmétique

Cette composition est préparée de manière classique pour l'homme du métier. Les quantités sont indiquées en pourcentages pondéraux.

| | | |
|---|---|---|
| Dihydrojasmonate de méthyle | 0,001% | |
| Acide n-octanoyl-5-salicylique | 1 | % |
| Méthylparaben | 0,1 | % |
| Propylparaben | 0,1 | % |
| Lanoline | 5 | % |
| Huile de vaseline | 4 | % |
| Huile de sésame | 4 | % |
| Alcool cétylique | 5 | % |
| Monostéarate de glycérol | 2 | % |
| Triéthanolamine | 1 | % |
| Propylène glycol | 5 | % |
| Carbomer 940 | 0,1 | % |
| Eau | qsp 100 | % |

Cette crème, utilisée en applications bi-quotidiennes, permet de raviver l'éclat et d'améliorer le confort des peaux sèches de femmes ménopausées.

## Revendications

1. Procédé cosmétique de traitement de la peau sèche ou du cuir chevelu sec d'origine non inflammatoire, d'une femme ménopausée, comprenant l'application topique sur la peau ou le cuir chevelu, d'une composition renfermant, dans un milieu physiologiquement acceptable, au moins un dérivé d'acide (dihydro)jasmonique choisi parmi les composés de formule (I): 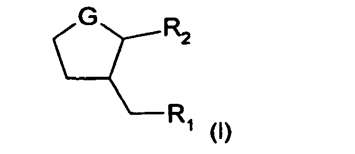 dans laquelle :
G désigne un groupe C=O ou CH-OH ; R₁ est un radical -COOR où R est un atome d'hydrogène ou un radical hydrocarboné comportant de 1 à 6 atomes de carbone, linéaire ou ramifié, saturé ou insaturé, non substitué ; R₂ est un radical hydrocarboné, linéaire ou ramifié, saturé ou insaturé, non substitué, comportant 5 atomes de carbone.

2. Procédé selon la revendication précédente, **caractérisé en ce que** R₁ est un radical - COOR où R est un radical méthyle ; R₂ est un radical n-pentyle.

3. Procédé selon la revendication 1 ou 2, **caractérisé en ce que** le dérivé d'acide (dihydro)jasmonique est le dihydrojasmonate de méthyle ou l'un de ses isomères, stéréoisomères et sels.

4. Procédé selon l'une quelconque des revendications précédentes, **caractérisé en ce que** le dérivé d'acide (dihydro)jasmonique représente de 0,5% à 5% du poids total de la composition.

5. Procédé selon l'une quelconque des revendications précédentes, **caractérisée en ce que** ladite composition renferme en outre au moins un composé choisi parmi : les agents desquamants ; les agents hydratants , les agents anti-inflammatoires ou apaisants ; les agents stimulant la prolifération et/ou la différenciation des kératinocytes ; les agents anti-chute des cheveux ; les agents anti-pelliculaires ; et les agents anti-bactériens.

6. Procédé selon l'une quelconque des revendications précédentes, **caractérisée en ce que** la composition est sous la forme d'une émulsion huile-dans-eau.

7. Procédé selon l'une quelconque des revendications précédentes, **caractérisé en ce que** la composition est appliquée sur des personnes présentant une sécrétion de sébum insuffisante, en particulier un taux de sébum inférieur à 100 µg/cm² au niveau du front.

8. Procédé selon l'une quelconque des revendications précédentes, **caractérisé en ce qu'**il est destiné à lutter contre l'aspect terne et/ou atone de la peau et/ou des cheveux.

9. Utilisation cosmétique d'au moins un dérivé d'acide (dihydro)jasmonique de formule (1), tel que défini dans l'une quelconque des revendications 1 à 3, en tant qu'agent pour le traitement des peaux sèches ou du cuir chevelu sec d'origine non inflammatoire de femmes ménopausées.

## Patentansprüche

1. Kosmetisches Verfahren zur Behandlung von trockener Haut oder trockener Kopfhaut nichtentzündlichen Ursprungs bei Frauen in den Wechseljahren, das das topische Aufbringen einer Zusammensetzung auf die Haut oder die Kopfhaut umfasst, die in einem physiologisch akzeptablen Medium mindestens ein (Dihydro)jasmonsäurederivat enthält, das unter den Verbindungen der folgenden Formel (I) ausgewählt ist: 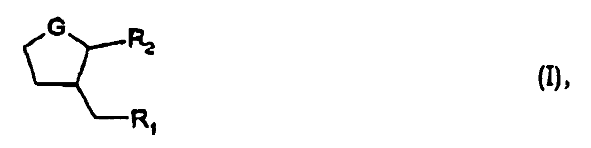 worin bedeuten:
G die Gruppe C=O oder CH-OH; R₁ eine Gruppe -COOR, worin R ein Wasserstoffatom oder eine geradkettige oder verzweigte, gesättigte oder ungesättigte, unsubstituierte Kohlenwasserstoffgruppe mit 1 bis 6 Kohlenstoffatomen bedeutet; und R₂ eine geradkettige oder verzweigte, gesättigte oder ungesättigte, unsubstituierte Kohlenwasserstoffgruppe mit 5 Kohlenstoffatomen.

2. Verfahren nach dem vorhergehenden Anspruch, **dadurch gekennzeichnet, dass** R₁ ein Gruppe -COOR bedeutet, worin R Methyl bedeutet; und R₂ die n-Pentylgruppe ist.

3. Verfahren nach Anspruch 1 oder 2, **dadurch gekennzeichnet, dass** es sich bei dem (Dihydro)jasmonsäurederivat um Methyldihydrojasmonat oder eines seiner Isomere, Stereoisomere und Salze handelt.

4. Verfahren nach einem der vorhergehenden Ansprüche, **dadurch gekennzeichnet, dass** das (Dihydro)jasmonsäurederivat 0,5 bis 5 % des Gesamtgewichts der Zusammensetzung ausmacht.

5. Verfahren nach einem der vorhergehenden Ansprüche, **dadurch gekennzeichnet, dass** die Zusammensetzung ferner mindestens eine Verbindung enthält, die ausgewählt ist unter: abschuppenden Wirkstoffen; Hydratisierungsmitteln; entzündungshemmenden Wirkstoffen oder beruhigenden Wirkstoffen; Wirkstoffen, die die Proliferation und/oder Differenzierung der Keratinocyten stimulieren; Wirkstoffen gegen Haarausfall; Wirkstoffen gegen Schuppen; und antibakteriellen Wirkstoffen.

6. Verfahren nach einem der vorhergehenden Ansprüche, **dadurch gekennzeichnet, dass** die Zusammensetzung als ÖI-in-Wasser-Emulsion vorliegt.

7. Verfahren nach einem der vorhergehenden Ansprüche, **dadurch gekennzeichnet, dass** die Zusammensetzung bei Personen angewandt wird, die eine ungenügende Sebumsekretion und insbesondere einen Sebumgehalt unter 100 µg/cm² an der Stirn aufweisen.

8. Verfahren nach einem der vorhergehenden Ansprüche, **dadurch gekennzeichnet, dass** es dazu vorgesehen ist, das glanzlose Aussehen und/ oder das atonische Aussehen der Haut und/ oder der Haare zu bekämpfen.

9. Kosmetische Verwendung mindestens eines (Dihydro)jasmonsäurederivats der Formel (I) nach einem der Ansprüche 1 bis 3 als Wirkstoff zur Behandlung von trockener Haut oder trockener Kopfhaut nichtentzündlichen Ursprungs bei Frauen in den Wechseljahren.

## Claims

1. Cosmetic process for treating dry skin or a dry scalp of non-inflammatory origin, in a menopausal woman, comprising the topical application to the skin or the scalp of a composition containing, in a physiologically acceptable medium, at least one (dihydro)jasmonic acid derivative chosen from the compounds of formula (I) : 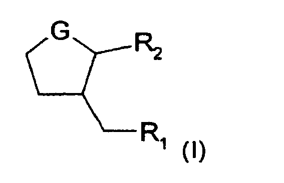 in which:
G denotes a group C=O or CH-OH; R₁ is a radical -COOR, where R is a hydrogen atom or an unsubstituted, saturated or unsaturated, linear or branched hydrocarbon-based radical containing from 1 to 6 carbon atoms; R₂ is an unsubstituted, saturated or unsaturated, linear or branched hydrocarbon-based radical containing 5 carbon atoms.

2. Process according to the preceding claim, **characterized in that** R₁ is a radical -COOR, where R is a methyl radical; R₂ is an n-pentyl radical.

3. Process according to Claim 1 or 2, **characterized in that** the (dihydro) jasmonic acid derivative is methyl dihydrojasmonate or one of its isomers, stereoisomers and salts.

4. Process according to any one of the preceding claims, **characterized in that** the (dihydro)jasmonic acid derivative represents from 0.5% to 5% of the total weight of the composition.

5. Process according to any one of the preceding claims, **characterized in that** said composition also contains at least one compound chosen from: desquamating agents; moisturizers; antiinflammatory agents or calmatives; agents for stimulating keratinocyte proliferation and/or differentiation; anti-hairloss agents; antidandruff agents; and antibacterial agents.

6. Process according to any one of the preceding claims, **characterized in that** the composition is in the form of an oil-in-water emulsion.

7. Process according to any one of the preceding claims, **characterized in that** the composition is applied to individuals exhibiting insufficient sebum secretion, in particular a sebum content of less than 100 µg/cm² on the forehead.

8. Process according to any one of the preceding claims, **characterized in that** it is intended to combat the dull and/or lifeless appearance of the skin and/or of the hair.

9. Cosmetic use of at- least one (dihydro)jasmonic acid derivative of formula (I) as defined in any one of Claims 1 to 3, as an agent for treating dry skin or a dry scalp of non-inflammatory origin in menopausal women.
